Europäisches Patentamt

European Patent Office

Office européen des brevets

(19)

(11) Veröffentlichungsnummer: **0 003 822**
**B1**

(12)

# EUROPÄISCHE PATENTSCHRIFT

(45) Veröffentlichungstag der Patentschrift:
15.09.82

(21) Anmeldenummer: 79100486.4

(22) Anmeldetag: 20.02.79

(51) Int. Cl.³: **A 61 B 5/00,** F 28 F 27/00,
G 05 D 23/19

(54) Vorrichtung zur Bestimmung von Blutungszeit und Blutungsverhalten einer kleinen standardisierten Wunde.

(30) Priorität: 28.02.78 DE 2808473

(43) Veröffentlichungstag der Anmeldung:
05.09.79 Patentblatt 79/18

(45) Bekanntmachung des Hinweises auf die Patenterteilung:
15.09.82 Patentblatt 82/37

(84) Benannte Vertragsstaaten:
CH FR GB NL SE

(56) Entgegenhaltungen:
DE-A-1 501 134
DE-A-1 566 651
DE-B-1 053 537
DE-B-1 272 409
US-A-3 191 391
US-A-3 293 868
US-A-3 888 259
US-A-3 967 627

(73) Patentinhaber: Hellige GmbH,
Postfach 728 Heinrich-von-Stephan-Strasse 4,
D-7800 Freiburg (DE)

(72) Erfinder: Fehlau, Robert, Valentinstrasse 16,
D-7800 Freiburg (DE)
Erfinder: Schlüssel, Peter, Goethestrasse 19,
D-7800 Freiburg (DE)
Erfinder: Schneider, Matthias, Dortustrasse 6,
D-7800 Freiburg (DE)

(74) Vertreter: Patentanwälte TER MEER - MÜLLER -
STEINMEISTER, Triftstrasse 4, D-8000 München 22 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Vorrichtung zur Bestimmung von Blutungszeit und Blutungsverhalten einer kleinen standardisierten Wunde

Die Erfindung betrifft eine medizinisch-diagnostische Vorrichtung zur Bestimmung von Blutungszeit und Blutungsverhalten einer kleinen standardisierten Wunde mit einer Einrichtung zur Konstanthaltung der Temperatur einer über diese Wunde geleiteten Flüssigkeit. Dieses diagnostische Verfahren hat die Bezeichnung »Hämorrhagometrie« erhalten. Durch den Strom der genannten Flüssigkeit, im nachfolgenden »Spülflüssigkeit« genannt, soll die standardisierte Wunde entweder auf eine vorbestimmbare Temperatur erwärmt oder gekühlt werden.

Mittels des medizinisch-diagnostischen Verfahrens der Hämorrhagometrie können gewisse Eigenschaften des Blutes und der Blutgefäße bestimmt werden, indem das zeitliche Verhalten der Blutung und die Blutungsintensität aus der standardisierten Wunde beobachtet wird. Es ist z. B. in der Veröffentlichung von A. H. Sutor u. a. »Bleeding from Standardized Skin Punctures«, erschienen in American Journal of Clinical Pathology, Vol. 55 (1971), Seite 541 ... 550, sowie in dem Bericht von A. H. Sutor »Die Hämorrhagometrie. Eine neue Invivo-Methode der Blutungsmessung«, erschienen in »Blut, Zeitschrift für die gesamte Blutforschung«, Band 32, Heft 3, Seite 157 ... 162, beschrieben. Vor allem sollen mit diesem Verfahren die Blutungszeit und die Blutgerinnungsfähigkeit bestimmt werden.

Anlaß für die vorliegende Erfindung hat die Erkenntnis gegeben, daß die Wundtemperatur auf das Blutungsverhalten maßgeblichen Einfluß hat. Man kann nämlich den diagnostischen Informationsumfang durch Herbeiführung bestimmter, vom normalen Wert abweichender Wundtemperaturen beträchtlich erweitern. Auf diese Weise kann ein sogenannter Kältebelastungstest oder eine sogenannte Wärmelysiszeitbestimmung durchgeführt werden, deren diagnostische Bedeutung in der zweiten der genannten Veröffentlichungen angegeben ist. Durch die erfindungsgemäße Ausstattung der vorgeschriebenen Vorrichtung zur Hämorrhagometrie mit einer Einrichtung für die Konstanthaltung der Temperatur (Thermostatisierung) der Spülflüssigkeit auf einen bestimmten einstellbaren Wert kann diesen diagnostischen Erfordernissen in fortschrittlicher Weise entsprochen werden.

Einrichtungen, um die Temperatur einer strömenden Flüssigkeit auf einem bestimmten Wert zu halten, sind auch schon im medizinischen Bereich bekannt. Vor allem ist in DE-A-1 566 651 eine zur Thermostatisierung eines extrakorporal zirkulierenden Blutstroms dienende Vorrichtung bekannt, womit eine Unterkühlung des Patientenkörpers (Hypothermie) erreicht werden soll. Diese Vorrichtung umfaßt bezüglich des Vergleichs mit der vorliegenden Erfindung drei Wärmeaustauscher, nämlich einen ersten Austauscher zwischen dem extrakorporal zirkulierenden Blut und einem Wärmeträger, einen zweiten Austauscher zwischen dem Wärmeträger und dem einen Pol (Wärmesenke) einer thermoelektrischen Batterie, die auch aus Peltier-Elementen zusammengesetzt sein kann, und einen dritten Wärmeaustauscher zwischen deren anderem Pol und einer Hilfsflüssigkeit; außerdem drei Temperaturfühler für die Regelung der Temperatur, wovon einer intrakorporal angeordnet ist, der zweite im extrakorporalen Blutkreislauf und der dritte im Ausfluß für den Wärmeträger am zweiter Wärmeaustauscher.

Die das Peltier-Element enthaltenden Wärme austauscher dieser bekannten Einrichtung habei im wesentlichen eine flache, scheibenförmige Gestalt und sind in bezug auf ihre Scheibenach se koaxial nebeneinander bzw. übereinande angeordnet.

Des weiteren ist in der US-Patentschrif 3 293 868 allgemein eine Kühleinrichtung fü Flüssigkeiten behandelt und auch ihre Anwen dung zur medizinischen Unterkühlung erwähn Dabei sind die Wärmeaustauscher und ei Peltier-Element in einem Gehäuse angeordne welches luftdicht abgeschlossen ist, und als ein alternative Ausführungsform ist die Wärmeau: tauschleitung spiralenförmig ausgelegt.

Bei den beschriebenen Einrichtungen solle offensichtlich große Volumina von Flüssigkeitei nämlich der gesamte Blutstrom, extrakorpor gekühlt bzw. auf einer bestimmten Temperatı gehalten werden. Dies erfordert eine aufwenc ge voluminöse Bauweise allein des Thermost ten, der somit eine eigene Geräteeinheit für d Therapie bzw. für die Patientenbehandlur bildet. Für das zuerst beschriebene Ger wurden zudem drei Wärmeaustauscher f erforderlich gehalten.

Anders als bei diesem bekannten Stand d Technik soll gemäß der vorliegenden Erfindu ein diagnostisches Gerät, nämlich ein Gerät die Hämorrhagometrie, mit einer zusätzliche möglichst kleinen, einfachen, aber wirksam Thermostatisierungseinrichtung für eine v gleichsweise geringe Durchflußmenge ei Spülflüssigkeit ausgestattet werden, damit Beschaffenheit des Blutes an einer klein standardisierten Wunde, die z. B. am A angebracht wird, bei verschiedenen Wundte peraturen bestimmt werden kann, wozu ledigl mittels einer laufend über die Wunde geleite Spülflüssigkeit diese Wunde auf einer vor stimmten Temperatur konstant gehalten wird.

Die vorliegende Erfindung, wie sie in Ansprüchen gekennzeichnet ist, hat diese A gabe gelöst. Die erfindungsgemäße Vorricht für die Hämorrhagometrie hat im wesentlic die vorteilhaften und fortschrittlichen ne Eigenschaften, daß sie nunmehr eine direkt die Erfordernisse des speziellen diagnostisc Verfahrens angepaßte, einfache, leichte, ra sparende und deshalb mit der eigentlic

photometrischen Meßeinrichtung und die sonstigen Organe ohne Schwierigkeit zu einem einzigen Gerät zusammenbaubare Einrichtung zur Thermostatisierung der Spülflüssigkeit umfaßt. Für die Anwendung im Routinebetrieb ist dies von großer praktischer Bedeutung. Es müssen nicht mehrere Geräteteile transportiert und dann am Einsatzort zusammengesetzt werden. Es muß auch kein großes Volumen der Spülflüssigkeit ständig auf der gewünschten Temperatur gehalten werden. Die durchlaufende Menge der Spülflüssigkeit, die als Temperaturträger für die kleine standardisierte Wunde wirkt, ist vergleichsweise gering, und deshalb ist auch für den schnellen Ausgleich von Temperaturschwankungen kein nennenswerter Energiebedarf für das Peltier-Element erforderlich. Die Thermostatisierungseinrichtung des Hämorrhagometriegerätes ist leicht zu reinigen und gut sterilisierbar. Für die Temperaturregelung bzw. -steuerung ist nur ein geringer technischer Aufwand erforderlich. Mit dem Peltier-Element können aber auch verhältnismäßig große Temperaturunterschiede bis zu mehreren Zehner-Graden Celsius leicht bewältigt werden, so daß Wundtemperaturen zwischen 5° Celsius und 50° Celsius für die verschiedenen hämorrhagometrischen Meßverfahren ohne Schwierigkeiten erreicht und laufend aufrechterhalten werden können.

Im folgenden wird die Erfindung anhand von zwei Figuren näher erläutert. Es zeigt

Fig. 1 schematisch die wesentlichen Organe eines Hämorrhagometriegerätes, das erfindungsgemäß mit einer Einrichtung für die Thermostatisierung der Spülflüssigkeit ausgestattet ist, welche Organe zu einem Kompaktgerät vereinigt werden können.

Fig. 2 in perspektivischer Darstellung eine Ausführungsform einer Einrichtung für die Thermostatisierung der Spülflüssigkeit.

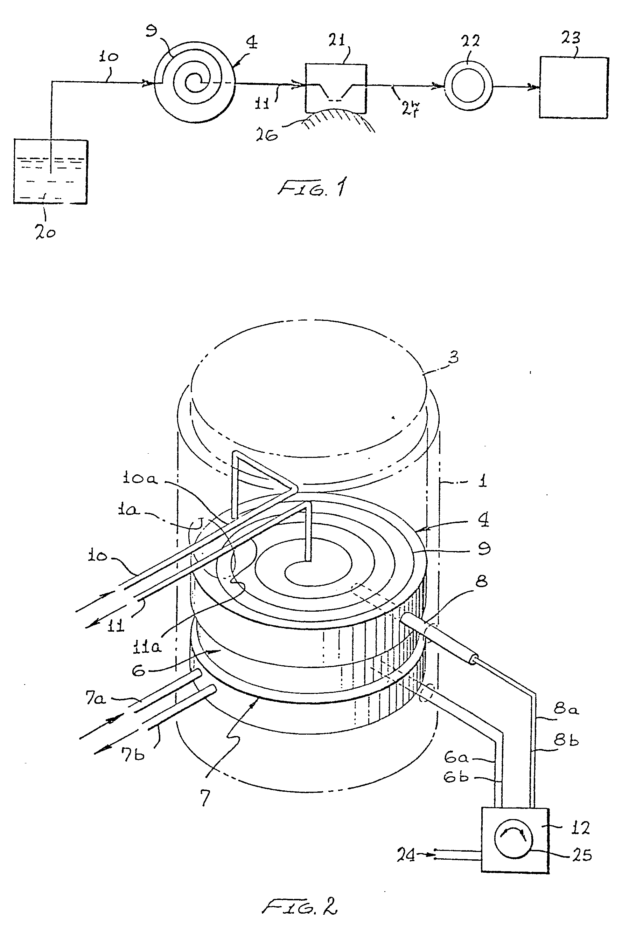

Bei einem Gerät für die Hämorrhagometrie wird, wie in Fig. 1 schematisch veranschaulicht, ein verhältnismäßig kleiner, mengenmäßig aber genau bestimmter Strom einer Flüssigkeit, hier destilliertes Wasser, über eine kleine, z. B. am Arm 26 der zu untersuchenden Person mittels eines Schneppers angebrachte, in bezug auf Größe und Tiefe standardisierte Wunde geleitet. Mittels eines Photometers 23 wird dann der Gehalt an mitgespültem Blut über eine gewisse Zeitdauer messend verfolgt und vor allem die Blutungszeit und das Blutungsverhalten festgestellt. Diese Kriterien sind diagnostisch höchst bedeutsam. Die Meßeinrichtung für die Hämorrhagometrie umfaßt einen Vorratsbehälter 20 für das destillierte Wasser, das daraus mittels einer kleinen Flüssigkeitspumpe 22, z. B. einer Schlauchpumpe, in bestimmten Mengen pro Zeiteinheit abgesaugt und über die Abflußleitung 10 dem Hauptaustauscher 4 des Thermostaten zugeleitet wird. Dort erhält der Wasserstrom laufend die vorgeschriebene bestimmte Temperatur und wird dann der Durchflußküvette 21 zugeleitet, die auf der Wunde angebracht wird, wobei jedoch der Durchflußkanal zur Wunde hi offen ist. Zwischen der Durchflußküvette un dem Photometer, das zur photometrische Bestimmung des Blutgehaltes im Flüssigkeits strom dient, ist die Förderpumpe 22 angeordne Zweckmäßig werden der Vorratsbehälter 20 fü das destillierte Wasser, der Thermostat 1, 4, di Förderpumpe 22 und das Photometer 23 und di anderen, hier nicht näher zu kennzeichnende Organe der Meßvorrichtung mit Ausnahme de Durchflußküvette 21 in einem einzigen Gehäus untergebracht bzw. zu einem Kompaktgerä zusammengefaßt. Die Durchflußküvette 21 is mit einer Zuflußleitung 11 und einer Abflußle tung 27 an dieses Kompaktgerät angeschlosse Analog wird auch der in Fig. 2 angegeben Hilfsaustauscher 7 mit der Hilfsflüssigkeit g€ speist.

Die wesentlichen Merkmale der Thermostat sierungseinrichtung des Gerätes für die Hämo rhagometrie gemäß der Erfindung sind in Fig. näher dargestellt. Die zu thermostatisierend Spülflüssigkeit wird durch eine Zuflußleitung 1 in den Hauptaustauscher 4 eingeleitet und durc die Abflußleitung 11 wieder hinausbeförder z. B. mittels einer geeigneten Flüssigkeitsförde pumpe 22 gemäß Fig. 1. Die Vorrichtung umfa weiterhin einen Hilfsaustauscher 7, überr we chen eine Hilfsflüssigkeit durch die Zuleitung ` und die Ausflußleitung 7b gefördert wird. Die Hilfsflüssigkeit soll durch geeignete, hier nic zu betrachtende Mittel, z. B. durch elektrisct Heizung oder elektrische Kühlung, auf ein Temperatur gehalten werden, der gegenüb das Peltier-Element 6 der Vorrichtung d Feinregelung der Temperatur für die Spülflüssi keit übernimmt, was keineswegs bedeuten mu daß nur noch ein geringer Unterschied zu gewünschten Wert der Temperatur ausgeg chen werden kann. Dieses Peltier-Element i zwischen dem Hauptaustauscher 4 und de Hilfsaustauscher 7 angeordnet, und zwar dera daß der eine Austauscher mit der Wärmesen des Peltier-Elementes und der andere Wärm austauscher mit der Wärmequelle des Pelti( Elementes einen möglichst guten wärmeleite den Kontakt haben, die Austauscher also d Peltier-Element möglichst großflächig, unmitt bar berühren. Dies gelingt am besten, wenn ( beiden Austauscher 4 und 7 und das Peltier-E ment 6 eine scheibenförmige flache Form hab< wie die Figur zeigt. Bekanntlich ist ein Pelti Element aus zwei metallischen Leitern zusa mengesetzt, zwischen denen sich ein Tempe turgefälle herausbildet, wenn ein elektrisct Strom durch diese Leiter und ihre Verbindun stelle hindurchgeschickt wird. Der eine met< sche Leiter stellt daher eine Wärmesenke c der andere Leiter eine Wärmequelle. Kehrt n die Stromrichtung um, tauschen sich auch Quelle und die Senke aus.

Ist also in der gezeigten Ausführungsform Spülflüssigkeit z. B. zu kühlen, dann ist elektrischer Strom durch das Peltier-Elemer über seine Zuleitungen 6a und 6b derart führen, daß der mit dem Hauptaustauscher 4 in wärmeleitendem Kontakt stehende metallische Leiter eine Wärmesenke wird und der andere Leiter, der mit dem Hilfsaustauscher 7 in Kontakt steht, als Wärmequelle wirkt. Wird die durch den Hilfsaustauscher 7 geleitete Hilfsflüssigkeit geeignet vorgekühlt, dann braucht die vom Peltier-Element gezeigte Temperaturdifferenz für eine Feinregelung nur noch klein zu sein. Aber auch ein verhältnismäßig großer Temperaturunterschied von mehreren Zehner-Graden Celsius könnte dabei bestehen und vom Peltier-Element bewältigt werden. Die durch den Hilfsaustauscher 7 fließende Hilfsflüssigkeit dient letzten Endes entweder im Falle der erforderlichen Kühlung der Spülflüssigkeit zur Abführung der überflüssigen Wärme bzw. bei notwendiger Erwärmung der Spülflüssigkeit zur Zuführung einer zusätzlichen Wärmemenge. Die Temperatur der Hilfsflüssigkeit kann deshalb in beiden Fällen höher oder tiefer liegen als die gewünchte Temperatur der Spülflüssigkeit. Die erforderliche Temperaturkorrektur wird in jedem Fall vom Peltier-Element bewirkt.

Grundsätzlich handelt es sich also um eine Vorrichtung zur Temperaturregelung, bei welcher die Temperatur der den Hauptaustauscher 4 verlassenden Spülflüssigkeit die Regelgröße darstellt, der Hauptaustauscher die Regelstrecke, der zur Speisung des Peltier-Elementes 6 dienende elektrische Strom die Stellgröße, und wozu noch der Regler 12 für den Peltierstrom benötigt wird. Die Stellgröße für den Regler wird von einem Temperaturfühler 8 geliefert, beispielsweise einem Thermoelement, der im Hauptaustauscher 4 möglichst dicht an der Ausflußleitung 11a für die Spülflüssigkeit angeordnet ist, damit er die Temperatur der ausfließenden Spülflüssigkeit messen kann. Der Regler 12 für den Peltier-Strom ist in Fig. 1 schematisch angedeutet. Zum Wählen des gewünschten Temperaturwertes dient der Schalter 25. Über den Leitungsanschluß 24 erhält der Regler 12 den Speisestrom für seine Organe und für das Peltier-Element 6. Der Temperaturfühler 8 ist mit dem Regler 12 über die Leitungen 8a, 8b verbunden. Die beschriebene Vorrichtung könnte selbstverständlich auch dann arbeiten, wenn die Funktionen des Hauptaustauschers 4 und des Hilfsaustauschers 7 vertauscht werden. Der Temperaturfühler müßte dann aber bei der Ausflußleitung 7b des Hilfsaustauschers angeordnet sein.

Es ist zweckmäßig, vor allem die Spülflüssigkeit möglichst unter Ausnutzung einer Art Gegenstromwirkung durch den Hauptaustauscher 4 zu führen. Dazu mündet der Zufluß 10a am äußeren Rande des scheibenförmigen Hauptaustauschers 4, und die Wärmeaustauschleitung in Form einer Kühlschlange oder Wärmeschlange 9 ist spiralig von außen nach innen bis zur Achse des Hauptaustauschers 4 geführt, wo sie an die Abflußleitung 11a angeschlossen ist. Im Laufe des Durchflusses durch diese Wärmeaustauschschlange erhält dann die Spülflüssigkeit durch den Kontakt mit dem Peltier-Element allmählich die gewünschte Temperatur, mit der sie die Vorrichtung verläßt. Für die Vorrichtung ist es daher günstig, wenn die scheibenförmigen Austauscher 4 und 7 und das scheibenförmige Peltier-Element 6 koaxial zu ihrer gemeinsamen Scheibenachse angeordnet sind.

Eine fertigungstechnisch und für die Reinigung zweckmäßige Gestaltung erreicht man, wenn die spiralförmige Wärmeaustauschleitung 9 von einer spiraligen Nute in der freien Oberfläche des Hauptaustauschers 4 gebildet wird. Da die einzelnen Spirallagen aber gegenseitig abgedichtet sein sollen, dient dazu der zylindrische Verschlußkörper 3, der auf der Seite des Hauptaustauschers in das Gehäuse 1 einsetzbar ist und dessen innere Stirnfläche bei Berührung mit der freien Oberfläche des Hauptaustauschers 4 mit dieser fluchtet und somit die einzelnen Arme der spiraligen Nute gegeneinander abdichtet. Bei dieser Ausbildung sind zweckmäßig die Anschlüsse 10a und 11a für den Zufluß und den Abfluß der Spülflüssigkeit in den Verschlußkörper 3 eingearbeitet, so daß bei einem richtigen Einsetzen des Verschlußkörpers unmittelbar die Verbindung zwischen der Wärmeaustauschleitung 9 und den genannten Anschlußleitungen hergestellt wird. Das winkelrichtige Einsetzen des Verschlußkörpers in das Gehäuse kann durch eine geeignete Führung erzwungen werden, z. B. durch eine Nockenführung. Der Verschlußkörper soll im Gehäuse einen gut passenden, wenigstens flüssigkeitsdichten Sitz haben, damit die zu thermostatisierende Spülflüssigkeit nicht teilweise beim Durchfluß durch den Austauscher 4 verlorengeht.

Die Wärmeaustauschleitung 9 kann auch von einem Schlauch, vorzugsweise aus Silikon-Kautschuk, gebildet werden, der in eine spiralförmige Nute in der freien Oberfläche des Hauptaustauschers 4 eingelegt ist. Dadurch wird ein direkter Flüssigkeitskontakt mit dem Austauscher auf die beiden Anschlußstellen reduziert und eher eine ungestörte Strömung der Spülflüssigkeit erzielt, was für die Hämorrhagometrie von Vorteil ist.

Um bei dieser Ausführungsform die Kopplung der Anschlußleitung 10 und 11 für den Zufluß und Abfluß der Spülflüssigkeit mit den Anschlußkanälen 10a und 11a im Verschlußkörper 3 zu gewährleisten, kann noch ein besonderes Kopplungsstück in Form eines Steckers vorgesehen sein, der durch die Öffnung 1a in eine Vertiefung am Mantel des Verschlußkörpers zu setzen ist, in welche die Anschlußkanäle 10a und 11a münden.

## Patentansprüche

1. Medizinisch-diagnostische Vorrichtung zur Bestimmung von Blutungszeit und Blutungsverhalten einer kleinen standardisierten Wunde (Hämorrhagometrie), mit einer zur Abdeckung dieser Wunde dienenden Durchflußküvette (21) für eine die Wunde überspülende Spülflüssigkeit, mit einer in die Durchflußküvette führenden Zuleitung (10) für die Spülflüssigkeit und mit einer ausgangsseitig an die Küvette angeschlossenen Abflußleitung (27), die über eine Förderpumpe (22) an eine photometrische Meßeinrichtung (23) angeschlossen ist, mittels welcher der Gehalt an Blut in der Spülflüssigkeit laufend bestimmbar ist, gekennzeichnet durch folgende Merkmale:

1.1 In die Zuflußleitung (10) ist vor der Durchflußküvette (21) eine zur Konstanthaltung einer bestimmten Temperatur der Spülflüssigkeit dienende Thermostatisiereinrichtung (1) eingefügt, deren Abflußleitung (11) zur Durchflußküvette (21) führt;

1.2 die Thermostatisiereinrichtung umfaßt
— in koaxialer Schichtanordnung einen flachen, scheibenförmigen Hauptaustauscher (4), einen flachen, scheibenförmigen Hilfsaustauscher (7) für Wärme und ein zwischen dem Haupt- und dem Hilfsaustauscher angeordnetes Peltier-Element 6, dessen Wärmesenke und Wärmequelle mit je einem der beiden Austauscher (4 bzw. 7) engen wärmeleitenden Kontakt hat,
— Anschlüsse (10a und 11a) für die Zuflußleitung (10) und die Abflußleitung (11) im Hauptaustauscher für die durch ihn geförderte Spülflüssigkeit, die auf einer bestimmten Temperatur zu halten ist,
— Anschlüsse (7a und 7b) am Hilfsaustauscher (7) für eine ihn durchströmende, zum Vorkühlen oder Vorwärmen dienende Hilfsflüssigkeit,
— einen im Hauptaustauscher (4) angeordneten Temperaturfühler (8), der über eine zugeordnete Vorrichtung (12) den das Peltier-Element (6) speisenden elektrischen Strom derart steuert, daß die Spülflüssigkeit auf einer bestimmten Temperatur konstant gehalten wird, und
— ein den aus Hauptaustauscher (4), Peltier-Element (6) und Hilfsaustauscher (7) bestehenden Schichtaufbau umschließendes Gehäuse, das auf der Oberseite des Hauptaustauschers (4) durch einen zylindrischen Verschlußkörper (3) luftdicht abgeschlossen ist, in den der Anschluß (10a) für die Zuflußleitung (10) für die zu kühlende Flüssigkeit zum Hauptaustauscher (4) und der Anschluß (11a) für die Abflußleitung (11) derart eingearbeitet sind, daß sie bei vollständig eingesetztem Verschlußkörper (3) den Anschluß an den Anfang und das Ende einer als spiralförmige Nut verlaufenden Wärmeaustauschleitung (9) herstellen, die in die freie Oberfläche des scheibenförmigen Hauptaustauschers (4) eingearbeitet und bei vollständig eingesetztem Verschlußkörper (3) dicht abgedeckt ist, so daß die einzelnen Spirallagen der Nut gegenseitig abgedichtet sind;

1.3 ein Vorratsbehälter (20) für die Spülflüssigkeit, die Thermostatisiereinrichtung (1), die Förderpumpe (22) und die photometrische Meßeinrichtung (23) sind in einem einzigen tragbaren Gerätegehäuse eingebaut.

2. Vorrichtung nach Anspruch 1, dadurch gekennzeichnet, daß die Wärmeaustauschleitung (9) als dünner Schlauch ausgeführt ist, der in die spiralförmig verlaufende Nut in der freien Oberfläche des Hauptaustauschers (4) eingelegt ist.

**Claims**

1. Medical diagnostic device for determining bleeding time and bleeding behaviour of a small standardized wound (haemorrhagometry), with a flow-through cuvette (21), arranged to close off the wound and to carry a rinsing liquid which rinses the wound and having an inlet conduit (10) leading into the flow-through cuvette for the rinsing liquid and an outlet conduit (27) connected at the outlet side of the cuvette and linked by means of a pump (22) to a photometric measuring instrument (23) by means of which the blood content of the rinsing liquid can be continuously determined, characterised by the following features:

1.1 In the inlet conduit (10), before the flow-through cuvette (21), a thermostat device (1), for holding a particular temperature of the rinsing liquid constant, is provided, the outlet conduit of which leads to the flow-through cuvette (21);

1.2 the thermostat device comprises
— in a coaxial alyered arrangement, a flat disc-shaped main heat exchanger (4) and a flat disc-shaped auxiliary heat exchanger (7), a Peltier effect element (6) being provided between said main and auxiliary heat exchangers and having its heat sink and heat source respectively each in intimate thermal contact with one of the two heat exchangers (4 or 7),
— connectors (10a and 11a) for the inlet conduit (10) and the outlet conduit (11) provided on the main heat exchanger for the rinsing liquid passing therethrough and which is to be maintained at a constant temperature,
— connectors (7a and 7b) on the auxiliary heat exchanger (7) for an auxiliary liquid, providing for pre-cooling or preheating, passing therethrough,
— a temperature sensor (8) provided in the main heat exchanger (4) which, via an associated device (12), controls the electrical current supplying the Peltier-effect element (6) in such a fashion that the rinsing liquid is maintained at a particular temperature, and

— a housing enclosing the layered structure made up by the main heat exchanger (4), the Peltier-effect element (6) and the auxiliary heat exchanger (7) and being closed off at the upper end of the main heat exchanger (4) in an airtight manner by a cylindrical closing lid (3) in which the connector (10a) for the inlet conduit (10) leading the liquid to be cooled to the main heat exchanger (4) and the connector (11a) für the outlet conduit (11) are machined in such a fashion that, upon complete insertion of the closing lid (3), they establish connection at the start and at the end of a heat exchange conduit (9) which follows the path of a spiral groove formed in the free surface of the disc-shaped main heat exchanger (4) and which is closed off in a sealed manner when the closing lid (3) is completely inserted so that the individual turns of the spiral groove are mutually sealed off;

1.3 a storage receptacle (20) for the rinsing liquid, the thermostat device (1), the pump (22) and the photometric measuring device (23) being incorporated in a single, portable apparatus housing.

2. Device in accordance with Claim 1, characterised in that the heat exchange conduit (9) takes the form of a thin tube which is inserted into the spiral groove in the free surface of the main heat exchanger (4).

**Revendications**

1. Dispositif médico-diagnostique pour la détermination de la durée de saignement et de l'allure du saignement d'une petite plaie normalisée (hémorragométrie), avec une cuvette de circulation (21) servant à recouvrir cette plaie, pour un liquide de rinçage rinçant la plaie, avec un conduit d'amenée (10) débouchant dans la cuvette de circulation, pour le liquide de rinçage, et avec un conduit d'évacuation (27) qui, raccordé à la cuvette côté sortie, est raccordé par une pompe de refoulement (22) à un dispositif de mesure photométrique (23), au moyen duquel la proportion de sang dans le liquide de rinçage peut être déterminée en permanence, caractérisé par les caractéristiques suivantes:

1.1 Dans le conduit d'amenée (10), avant la cuvette de circulation (21), se trouve un dispositif de thermostatisation (1) servant à maintenir constante une température déterminée du liquide de rinçage, dont le conduit d'évacuation (11) mène à la cuvette de circulation (21);

1.2 Le dispositif de thermostatisation comprend:

— en un agencement par couches coaxiales, un échangeur principal (4) plat et discoïdal, un échangeur auxiliaire (7) plat et discoïdal pour la chaleur et un élément Peltier (6), intercalé entre l'échangeur principal et l'échangeur auxiliaire, dont le puits thermique et la source de chaleur ont un contact intime thermoconducteur avec l'un respectif des deux échangeurs (4 ou 7),

— des raccordements (10a et 11a) pour le conduit d'amenée (10) et le conduit d'évacuation (11), dans l'échangeur principal, pour le liquide de rinçage le traversant, qui doit être maintenau à une température déterminée,

— des raccordements (7a et 7b) sur l'échangeur auxiliaire (7), pour un liquide auxiliaire qui le parcourt et qui sert au refroidissement préalable ou au chauffage préalable,

— une sonde de température (8) qui, disposée dans l'échangeur principal (4), commande, par l'intermédiaire d'un dispositif associé (12), le courant électrique alimentant l'élément Peltier (6), de telle sorte que le liquide de rinçage soit maintenu en permanence à une température déterminée, et

— un boîtier qui, entourant l'agencement par couches comprenant l'échangeur principal (4), l'élément Peltier (6) et l'échangeur auxiliaire (7), est hermétique à l'air à la face supérieure de l'échangeur principal (4), par un corps cylindrique d'obturation (3) dans lequel le raccord (10a) pour le conduit d'amenée (10) à l'échangeur principal (4) du liquide à refroidir, et le raccord (11a) pour le conduit d'évacuation (11) sont ménagés de telle manière que, lorsque le corps d'obturation (3) est totalement inséré, ils forment le raccordement au début et à la fin d'un conduit d'échange thermique (9) en forme de gorge spiroïdale, qui est ménagé dans la face supérieure libre de l'échangeur principal discoidal (4) et qui est recouvert de manière étanche, lorsque le corps d'obturation (3) est totalement inséré, de façon que les couches spiroïdales individuelles de la gorge soient mutuellement rendues étanches;

1.3 Un réservoir (20) pour le liquide de rinçage, le dispositif de thermostatisation (1), la pompe de refoulement (22) et le dispositif de mesure photométrique (23) sont incorporés dans un seul et unique boîtier d'appareil portatif.

11 **0 003 822**

2. Dispositif solon la revendication 1, caractérisé par le fait que le conduit d'échange thermique (9) est réalisé sous la forme d'un tuyau mince, qui est inséré dans la gorge spiroïdale dans la face supérieure libre de l'échangeur principal (4).

FIG. 1

FIG. 2

9